(19) Europäisches Patentamt
European Patent Office
Office européen des brevets

(11) Publication number: 0 007 120
B1

(12) # EUROPEAN PATENT SPECIFICATION

(45) Date of publication of patent specification: 16.02.83

(51) Int. Cl.$^3$: A 61 K 7/50, C 11 D 7/24

(21) Application number: 79200297.4

(22) Date of filing: 08.06.79

(54) ## Handwashing composition and a process for its preparation.

(30) Priority: 30.06.78 NL 7807077

(43) Date of publication of application:
23.01.80 Bulletin 80/2

(45) Publication of the grant of the patent:
16.02.83 Bulletin 83/7

(84) Designated Contracting States:
AT BE CH DE FR GB IT LU NL SE

(56) References cited:
BE - A - 808 588
CH - A - 283 429
CH - A - 375 820
DE - A - 1 934 913
DE - A - 2 646 434
DE - C - 647 523
FR - A - 1 196 246
FR - A - 1 501 837
FR - A - 2 097 333
FR - A - 2 104 997
FR - A - 2 208 644
FR - A - 2 262 692

(73) Proprietor: Chemisch Adviesbureau Drs. J.C.P.Schreuder
P.O. Box 430
NL-3740 AK Baarn (NL)

(72) Inventor: Schreuder, Johannes Carolus Petrus
Veenbesstraat 712
NL-3765 BV Soest (NL)

(56) References cited:
FR - A - 2 302 775
FR - A - 2 326 914
GB - A - 763 102
GB - A - 844 929
US - A - 2 118 566
US - A - 3 634 265
US - A - 3 659 025

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 007 120 B1

Courier Press, Leamington Spa, England.

# Handwashing composition and a process for its preparation

The invention is relating to a handwashing composition, to a process for its preparation and to the application of such composition.

Already several means and systems have been proposed during the last decades for an easy and efficient cleaning of particularly hands which are often and chronically exposed to filthiness on account of technical and more particularly industrial processes.

For example, handwashing compositions were known from the British Patent No. 763.102, containing

— mineral oil as a continuous oil phase
— a dispersed or emulsified aqueous phase
— an emulsifying system comprising partial esters of the higher natural fatty acids and glycerol, and ethoxylated gliceryne esterified by fatty acids, and
— other conventional agents.

Also the French Patent No. 1196.246 described handwashing compositions containing

— vaseline oil
— polyethyleneglycol stearate
— triethanolamine
— a polysaccharide, and
— water.

From the US Patent No. 3,659,025 skin cleaning compositions were known containing

— mineral oil
— triethanolamine
— a polysaccharide and
— water,

while in the US Patent No. 2118566 buffer systems for cosmetics are used to maintain the normal acidity of the skin.

However, with these previously proposed systems, the desired effect could not be reached.

In general, this was attributed on the one hand to the fact that one was starting from oil in water emulsions when composing of such means or systems, i.e. starting from a dispersed oil or fat phase in a continuous matrix of an aqueous phase, whereby the desired taking up of filthiness by the cleaning agent did not at all or not sufficiently take place.

On the other hand, the desired effect was presumed not to be reached too, on account of the proposed water in oil emulsions not being sufficiently long stable for an adequate application, caused by emulsifying systems being not or less suitable.

Another disadvantage of the systems and means proposed up to now, was the necessity to wash away the dirt with water, whereby the naturally occurring protecting components of the skin tissue are regularly washed away; which is giving rise, certainly on the long term, to several more or less serious affections of the skin, such as for example dermatitis or dermatose.

On account of this there still remains a growing need of cleaning agents for a skin which is regularly exposed to an intensive filthiness, caused by a large number of different acts in the field of industry in the widest sense, such as the filthiness occurring by working with paints, lacquers, glues, adhesives, printing inks, metal compositions, rusty metal accessories of for example motor cars, cement and the like or caused by regular contact with disease germs containing stuff.

Surprisingly an improved hand washing composition was found, which is composed of at least the following characterizing ingredients:

—a continuous oil phase, mainly consisting of straight or branched paraffinic oil fractions of 10—30 carbon atoms in the chain and preferably 12—25 carbon atoms, in an amount of 20—60% by weight and preferably of 25—40% by weight, based on the total system, whereby the paraffinic oil fractions having a boiling range between 140—400°C and have a viscosity of up to 35 centistokes at 25°C,

—a dispersed aqueous phase (emulsified phase)

—a buffer, mainly composed by lactic acid and triethanolamine giving rise to the normal pH of the outer skin tissue layers.

The lactic acid, preferably in the natural occurring L(+) optical isomer, is present in a percentage by weight, based on the total system of 0.1—1.5% and preferably 1%, while the triethanolamine is present in a percentage by weight, calculated with reference to the total system of 1—2% by weight and preferably 1.5%.

The amounts of lactic acid and triethanolamine are preferably selected in such a way, that a pH of about 5—6 is reached.

Such a buffer system appeared to be very suitable to supplement or even to replace temporarily the buffer system naturally occurring in the outer skin layers.

—an emulsifying system, mainly consisting of mono- and diglycerides of the higher natural fatty acids such as linoleic acid, oleic acid, palmitic acid, lauric acid or combinations thereof, and ethoxylated glycerine, esterified by fatty acids, according to the general formula:

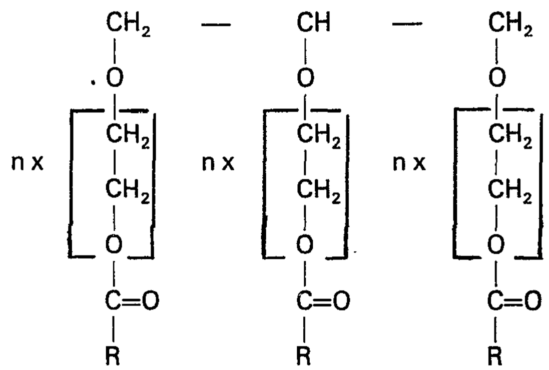

1

wherein n represents a number between 20 to 45 and preferably between 30 and 35, while R represents a saturated or unsaturated fatty acid residue derived from animal or vegetable oils, such as palmitic acid, lauric acid, oleic acid or linoleic acid, whereby R represents the same or different fatty acid residues in one molecule, but preferably the same (e.g. Tagat TO®).

In contrast to a large number of other similar systems which were applied for this purpose, such an emulsifying system surprisingly appears to meet very well on the one hand the requirement, stipulating that not too large amounts of the decidedly indispensable emulsifying agents may be used with reference to occurring skin irritations, as only relatively small amounts appear to be necessary as compared with those amounts of the formerly proposed systems, while on the other hand the emulsifying system applied according to the present invention may be regarded as especially affable to the skin, which feature is connected with a relatively low hydrophil-lipophil balance value.

The ratio between on the one hand the amount of the mono- and diglycerides and on the other hand the ethoxylated triglycerides according to formula 1 may vary, while the advantageous characteristics are maintained from 10—100 parts of mono- and diglycerides pro part of ethoxylated triglycerides and preferably about 25 parts of the mono- and diglycerides pro part of ethoxylated triglycerides.

The total percentage by weight of the beforementioned emulsifying system, calculated on the weight of the total system may vary from 1—5%, but will preferably be 3% by weight for the most optimal results.

It will be appreciated without any doubt, that the presently needed amount of the proposed emulsifying agent is significantly smaller than those according to the formerly proposed emulsifying systems, namely about five times smaller, which may be regarded as an important and actual advantage of this emulsifying system.

—Glycerine, as a stabilizer of the emulsion in a percentage by weight, calculated on the weight of the total system, of 0.5—5.0% and preferably 2% by weight, while the experimentally found attractive results possibly may be explained by presuming an additional function of the glycerin, which might consist of a moisture regulating action, optionally in interaction with the aforementioned lactic acid—triethanolamine combination, which also might cause an additional advantageous effect.

—caraghenates, i.e. polysaccharides bearing sulphonic acid residues and preferably those of natural origin, such as those derived from seaweeds, suitable for that purpose. The sulphonic acid residues may optionally be esterified by glycol, propylene glycol and glycerol (modified carraghenates).

Such carraghenates experimentally appeared to effect a surprisingly attractive stabilizing and film forming effect of the total system, to be applied on the skin, while as an additional advantageous effect, the known attractive properties of these caraghenates, such as the elimination of an eventual hardening of sore tissue and the therewith connected curing without or with less extensive scars, as well as the advantageous healing effect and the complex forming properties with proteins and/or metal ions, appeared to be maintained in the final, total system.

In connection therewith, the cleaning effect is also strongly improved on account of the easier elimination of e.g. polluting undesired metal ions in a complexed state.

It appeared that the caraghenates have to be added in an amount of 0.1—2% by weight, calculated on the weight of the total system and preferably 1.0%.

Moreover it was found, that in addition to the indicated primary basic components some additional secondary components have to be added to reach the most optimal results, such as e.g.:

—allantoin, the facilitation of curing of the skin by which is known from prior art, in an amount of 0.1—2% by weight, calculated on the weight of the total system and preferably 0.2%.

—additional stabilizers such as for example systems consisting of montmorillonites, the free oxygen sites of which are occupied by quaternary groups. Examples of such systems, which are preferably applied in the presently proposed systems are e.g. the so called Bentones® and Propaloids® in an amount of 0.7—2% by weight, calculated on the weight of the total system and preferably 1.0%.

Now it was surprisingly found that in the systems of the present invention with the relatively low concentration of these quaternary modified montmorillonites, not any falling out of one or more of the components appeared to occur and particularly not in the relatively low viscous systems, which are preferably used for practical reasons.

—lower alkanol, for a fast gelation of the quaternary montmorillonites.

The alkanol may be added in an amount of 0.1—1% by weight and preferably in an amount of half the amount of the montmorillonites.

—For special applications, e.g. in cases of intensive filthiness, an abrasive e.g. in the form of quartz powder and silver-sand and preferably silver-sand up to an amount of 5% by weight and preferably 2—2.5% by weight may be added, if necessary.

—Preservatives, e.g. esters of p-hydroxybenzoic acid, and for instance Nipagen® and Nipasol® in an amount of 0.02—0.08% by weight, calculated on the total weight and preferably 0.05% by weight with reference to the total weight and in a percentage in the aqueous phase of 0.05—0.2% by weight and preferably 0.1%, calculated on the total system.

Preferably the propylester of p-hydroxybenzoic acid is added to the oil phase and the methylester of p-hydroxybenzoic acid is added to the aqueous phase.

As unexpected advantageous effect was found, that the indicated composition of basic ingredients appeared to be much less sensitive to growing of bacteria and moulds, on account of which significantly smaller amounts of preservative were sufficient, as compared with those in the formerly usual oil in water emulsions.

—Silicone oils, preferably showing a viscosity of ⩾100 cps, in connection with the dirt repelling properties as known from literature, with reference to which the filthiness of the outer skin tissue is occurring less fast.

The silicone oils are occurring up to a content of about 0.5—3% by weight and preferably 1.5% calculated on the total weight of the mixture,

—eventually small amounts of antioxydants may be added up to an amount of 0.01—0.3%.

—perfumes in an amount of from 0.03—0.3% by weight.

—desinfectants, such as hexachlorophene or Irgasan DP 300® in an amount up to 1.5% by weight.

As compared with the formerly proposed hand washing agents for the defrayment of especially the filthiness on account of much occurring acts in the industry, the present compositions are characterized by a relatively low viscosity and high stability, as well as at high as at low temperatures, and show the following significant practical advantages:

—The skin tissue generally remains in a better condition, as essential outer skin ingredients are not regularly washed away with water.

—No chance or a much smaller chance to determatitis with a prolonged use and no irritation on account of the selected environmentally affable ingredients.

—For the use of the present composition not any water is needed, which means in several much occurring situations a practical advantage with reference to non-availability of water.

Fast and efficient cleaning action, caused by the dissolving power of the oil phase for a lot of pollutions such as paints, glues, and the like, which has a direct contact with the outer skin tissue and which penetrates as it were under the dirt.

—Only small amounts are necessary for obtaining the desired effect. For example about 200 sufficient cleanings of intensively filthy hands are possible per liter of the presently proposed agent.

It will be appreciated that the attractive properties of the presently proposed handwashing compositions could not be predicted or expected by people skilled in the art on account of the disclosed subject matter of the beforementioned GB—763.102, FR—1196246, US—3659025 and US—2118566.

The hand washing agents of the indicated composition may be prepared according to methods, which are usual for such preparations, whereby however the sequence of the addition and the dosing rate of the respective ingredients and for example also the temperature to be important.

The hereinbefore proposed handwashing creams may for example be prepared by the gelation of e.g. the montmorillonite fraction in the oil phase, to which the emulsifying agent has previously been added, at a temperature of 50—70°C and preferably 55—65°C, optionally by means of the addition of a lower alkanol up to an amount of at most half of the weight of the montmorillonite fraction e.g. Bentones®, whereafter the preservative and optionally one or more of the antioxydants are dissolved into the obtained dispersion, whereby the preservative and/or the antioxydants eventually may also be

added in a previous stage. With the term "lower alkanol" is meant an alkanol having 1—4 carbon atoms.

If desired, the obtained mixture is cooled to a temperature of preferably 40°C, whereafter the silicone oil is added, the silicone oil may also be added in a previous stage of the process. All other ingredients (glycerine, lactic acid, triethanolamine, allantoine, caraghenates, methylester of p-hydroxybenzoic acid) are dissolved into the aqueous phase, whereafter the mixture optionally may be cooled to a temperature of 35—40°C.

The phases are thoroughly mixed by means of a stirrer during 10—30 minutes and preferably 20 minutes and are subsequently homogenized until a size of the dispersed particles $<5\mu$ and preferably $<3\mu$ is reached, whereafter the eventual abrasive (silver-sand) may be added under thoroughly stirring.

It will be appreciated, that the present process is surprisingly characterized by a striking simplicity in contrast to those of most of the formerly proposed water-in-oil emulsions, most of which could only be homogenized by means of rolling.

Moreover the present process may be regarded as saving labour and energy, which means a decrease of the cost price.

It will be appreciated that the treatment of filthy skin parts with one of the hand washing compositions according to the present invention also has to be regarded as one of the features of the invention.

Such a treatment is preferably carried out by a process, characterized by the application of a small amount of the composition in an amount of from 2—10 ml and preferably about 6 ml of the concerning skin part, followed by thoroughly rubbing in, whereby the time range is dependent on the kind of filthiness, whereafter the skin parts are rubbed clean and dry with a cloth or tissue.

An additional advantage of such a treatment is situated in the absence of a greasy skin surface immediately after the treatment, e.g. papers or documents may be touched almost immediately after treatment.

The following examples are illustrating the hand washing compositions and their preparation, as well as the results obtained by their application according to the present invention, however, without restricting this invention in any way.

Example I

Composition 1

| | | | |
|---|---|---|---|
| Paraffinic oil I (Shell Ondina 15®) (b.p. 295—390°C) | | 150 g | oil phase |
| Paraffinic oil II (Shellsol T®, b.p. 176—211°C) | | 150 g | |
| Mono- and diglycerides | | 20 g | |
| Ethoxylated triglycerides | | 0.2 g | |
| Propyl p-hydroxybenzoate | | 0.5 g | |
| Antioxydants (2,6-di-tert. butyl p-kresol) | | 0.25 g | |
| Silicone oil 500 cps | | 10 g | |
| quaternary modified (Bentones®) montmorillonites | | 8 g | |
| iso butanol | | 4 g | |
| water | q.s. | 1000 g | aqueous phase |
| glycerine | | 40 g | |
| lactic acid (50%, L(+)) | | 20 g | |
| triethanolamine (85%) | | 20 g | |
| allantoin | | 2 g | |
| carraghenate | | 10 g | |
| methyl-p-hydroxybenzoate | | 1 g | |
| silver sand | | 20 g | |

Composition 2

| | | | |
|---|---|---|---|
| paraffinic oil I | | 500 g | oil phase |
| mono- and diglycerides | | 40 g | |
| ethoxylated triglycerides (Tagat TO®) | | 4 g | |
| propyl p-hydroxybenzoate | | 0.5 g | |
| antioxydants | | 0.2 g | |
| silicone oil (1000 cps) | | 5 g | |
| quaternary modified montmorillonites | | 16 g | |
| ethanol | | 4 g | |
| water | q.s. | 1000 g | aqueous phase |
| glycerine | | 20 g | |
| lactic acid (50%, L(+)) | | 10 g | |
| triethanolamine (85%) | | 10 g | |
| allantoin | | 2 g | |
| caraghenate | | 5 g | |
| methyl p-hydroxybenzoate | | 1 g | |

The beforementioned compositions are prepared as follows:

The oil phase is heated to 55—65°C and all occurring components are dissolved.

The quaternary modified montmorillonites are dispersed and subsequently gelated by the addition of the lower alkanol. Hereafter there is cooled to about 40°C.

In the aqueous phase of a temperature of 35—40°C the hereinbefore indicated ingredients are added after each other in the indicated order and dissolved.

The phases are thoroughly mixed and the emulsion is cooled to room temperature. In addition the mixture is homogenized, and if desired an abrasive is added under thoroughly stirring.

Example II

A handwashing composition is prepared according the process described in Example I and is containing

| | | | | |
|---|---|---|---|---|
| paraffinic oil I | | 17% by weight | | oil phase |
| paraffinic oil I | | 17% | | |
| paraffinic oil III (Shellsol K®, b.p. 193—245°C) | | 10% | | |
| mono- and diglycerides } ethoxylated triglycerides } | | 3% | | |
| propyl p-hydroxybenzoate | | 0.05% | | |
| antioxydants | | 0.02% | | |
| quaternary modified montmorillonites (Bentones®) | | 1.0% | | |
| propanol | | 0.5% | | |
| | | by weight | | aqueous phase |
| water | q.s. | 100% | | |
| glycerine | | 2% | | |
| lactic acid (50%, L(+)) | | 2% | (i.e. 1.0%) | |
| triethanolamine (85%) | | 1.4% | (i.e. 1.2%) | |
| allantoin | | 0.2% | | |
| modified caraghenate | | 1% | | |
| methyl p-hydroxybenzoate | | 0.1% | | |
| perfume | | 0.05% | | |

Example III

A handwashing composition is prepared according to the process described in Example I and is containing

| | | | | |
|---|---|---|---|---|
| | | by weight | | |
| paraffinic oil I | | 15% | | oil phase |
| paraffinic oil II | | 15% | | |
| mono- and diglycerides | | 2% | | |
| ethoxylated triglycerides | | 0.02% | | |
| propyl p-hydroxybenzoate | | 0.05% | | |
| antioxydants | | 0.02% | | |
| silicone oil (500 cps) | | 1% | | |
| quaternary modified montmorillonites | | 1.8% | | |
| ethanol | | 0.9% | | |
| | | by weight | | |
| water | q.s. | 100% | | aqueous phase |
| glycerine | | 4% | | |
| lactic acid | | 2% | | |
| triethanolamine | | 2% | | |
| allantoin | | 0.2% | | |
| caraghenate | | 1% | | |
| methyl p-hydroxybenzoate | | 0.1% | | |
| silver sand | | 2% | | |
| perfume | | 0.1% | | |

Example IV

A handwashing composition is prepared according to the process described in Example I and is containing

| | | | | |
|---|---|---|---|---|
| | | by weight | | |
| paraffinic oil I | | 20% | | oil phase |
| paraffinic oil III | | 18% | | |
| mono- and diglycerides }<br>ethoxylated triglycerides } | | 2% | | |
| propyl p-hydroxybenzoate | | 0.03% | | |
| silicone oil (100 cps) | | 1.5% | | |
| quaternary modified montmorillonites (Bentones®) | | 0.8% | | |
| propanol | | 0.4% | | |
| | | by weight | | |
| water | q.s. | 100% | | aqueous phase |
| glycerine | | 2% | | |
| lactic acid (50%, L(+)) | | 3% | (i.e. 1.5%) | |
| triethanolamine (85%) | | 2.1% | (i.e. 1.8%) | |
| allantoin | | 0.3% | | |
| caraghenate | | 1% | | |
| methyl p-hydroxybenzoate | | 0.1% | | |
| perfume | | 0.08% | | |

Example V

A handwashing composition is prepared by heating under vigorously stirring about 150 g of the paraffinic oil I to 55—60°C and the following oil phase components are dissolved:

20 g of mono- and diglycerides,
1 g of ethoxylated triglycerides,
0.2 g of antioxydant and
0.5 g of propyl p-hydroxybenzoate

Stirring is continued and the quaternary modified montmorillonites (10 g) are dispersed and

subsequently gelated, while a temperature is kept between 55—65°C. Subsequently 180 g of the paraffinic oil I are added and the obtained mixture is cooled to about 40°C.

An aqueous phase is prepared at a temperature of 35—40°C, containing
40 g of glycerine,
20 g of lactic acid (50%),
20 g of triethanolamine (85%)
2 g of allantoin,
10 g of modified caraghenate and
1 g of methyl-p-hydroxybenzoate
in about 545 g of water by the addition of all ingredients under stirring until complete dissolution.

Both obtained phases are thoroughly mixed and the emulsion is cooled to room temperature.

The mixture is homogenized by thoroughly stirring. The application of the handwashing compositions give rise to the following advantageous effects:

- — Cement hands are well cleaned. $CrO_3$ residues, residing in the pores are removed by the present compositions in contrast to the known compositions.
- — Hands polluted by repairs to a silencer are well cleaned.
- — Scalds by the silencers, occurring with these repairs, seemed less severe and healed faster after cleaning with the present handwashing compositions.
- — Printing inks, stencil inks and paints may very well be removed, as well as latex paints and glues.
- — After treatment the hands are soft and smooth and certainly not dry, wherethrough on the one hand repelling of dirt is reached and on the other hand chances on infection are decreased, as the natural barrier of skin fats and sweat is not completely eliminated by these compositions, but is supplied.

## Claims

1. Handwashing composition, characterized in that is composed of at least the following ingredients:

- — a continuous oil phase, mainly consisting of straight or branched paraffinic oil fractions of 10—30 carbon atoms in the chain in an amount of 20—60% by weight based on the total composition.
- — a dispersed aqueous phase (emulsified (phase)
- — a buffer, mainly composed by lactic acid in an amount of 0.1—1.5% by weight based on the total composition and triethanolamine in an amount of 1—2% by weight of the total composition, the buffer giving rise to the normal pH of the outer skin tissue layers
- — an emulsifying system in an amount of 1—5% by weight based on the total composition, namely consisting of mono- and diglycerides of the higher natural fatty acids, and ethoxylated glycerine, esterified by fatty acid, according to the general formula

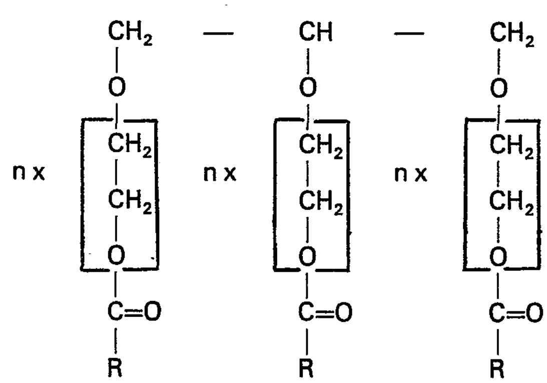

wherein n represents a number between 20 to 45 and preferably between 30 and 35, while R represents a saturated or unsaturated fatty acid residue derived from animal or vegetable oils, whereby R represents the same or different fatty acids residues in one molecule, the ratio of the amounts of the mono and diglycerides and ethoxylated triglycerides varying between 10—100 parts of mono- and diglycerides pro part of ethoxylated triglyceride

- — glycerine in an amount of 0.5—5% by weight based on the total composition
- — caraghenates, optionally modified by esterification of the sulphonic acid residues by glycol, propyleneglycol and glycerol in an amount of 0.1—2% by weight, calculated on the total composition.

2. Handwashing composition according to claim 1, characterized in that this composition contains stabilizing agents such as systems consisting of montmorillonites, the free oxygen sites of which are occupied by quaternary groups.

3. Handwashing composition according to claims 1 and 2, characterized in that this composition contains as additional ingredients allantoin, propyl-p-hydroxybenzoate and/or methyl-p-hydroxybenzoate as preservatives, silicone oil, antioxidants, a lower alkanol containing 1 to 4 carbon atoms, perfumes and desinfectants.

4. Handwashing composition according to claim 1, characterized in that the continuous oil phase, consisting of optionally branched paraffinic oil fractions of 12—25 carbon atoms, is present in a percentage by weight of 20—60% and preferably 25 to 40% by weight of the total composition; the buffer consists of lactic acid in an amount of 1.0% by weight, calculated on the weight of the total system, while the triethanolamine is present in an amount to give rise to a pH of 5—6 and whereby the ratio between the amount of mono- and diglycerides and the ethoxylated triglycerides according to formula 1 is 25 parts of mono- and diglycerides pro part of ethoxylated triglycerides, in a total percentage, calculated on the weight of the total system of about 3% by weight; glycerine is present in a percentage of 2% by weight, the carraghenates in a percentage of 1.0% by weight and a lower alkanol in a percentage by weight of 0.1—1%, calculated on the weight of the total system.

5. Handwashing composition according to claims 1—4, characterized in that it contains allantoin in an amount of 0.1—2% by weight and preferably about 0.2% calculated on the weight of the total mixture; montmorillonites in an amount of at most 2% by weight and preferably 1.0% calculated on the total mixture, preservatives in the form of esters of para hydroxybenzoic acid in an amount of 0.02—0.08% by weight in the oil phase and preferably 0.05% by weight and in an amount in the aqueous phase of 0.05—0.2% by weight and preferably 0.1%, calculated on the weight of the total system; silicone oil having a viscosity of $\geqslant$100 cps up to an amount of 3% by weight, calculated on the total weight of the composition, one or more antioxidants in an amount of 0.01 to 0.03% by weight, perfumes in an amount of 0.03—0.3% by weight, and desinfectants in an amount up to 1.5% by weight calculated on the weight of the total system.

6. Handwashing composition according to claims 1—5, characterized in that the applied emulsifying system is composed of mono- and diglycerides of oleic acid and ethoxylated glycerine, esterified by oleic acid according to formula 1, wherein n represents a number between 30 and 35.

7. Handwashing composition according to claim 3, 5 and 6, characterized in that as preservative in the oil phase the propyl ester of p-hydroxybenzoic acid is present in an amount of 0.02—0.05% by weight and as preservative in the aqueous phase the methyl ester of p-hydroxybenzoic acid is present in an amount of 0.05 to 0.1% by weight calculated on the total system.

8. Process for the preparation of a handwashing composition according to the preceding claims 1—7, characterized in that the fatty or oil phase is mixed with the emulsifying system, optionally followed by the preservative and/or the antioxidants and/or silicone oil, followed by the addition and gelation of the montmorillonite fraction, at a temperature of from 50—75°C and preferably 55—65°C, followed by the addition of lower alkanol up to an amount of at most half of the weight of the montmorillonite fraction, optionally followed by the addition of the preservative and/or antioxidants, optionally followed by the addition of the silicone oil, followed by the addition of the aqueous phase, wherein all other ingredients previously have been dissolved at about 40°C, optionally followed by interim, vigorous stirring during 10—30 minutes and followed by homogenizing until a size of the dispersed particles of $<5\mu$ and preferably $<3\mu$ is reached, and whereafter an abrasive is optionally added under vigorous stirring.

**Patentansprüche**

1. Handwaschmittel, gekennzeichnet durch mindestens folgende Bestandteile:

— eine kontinuierliche Ölphase, hauptsächlich bestehend aus Fraktionen von unverzweigten oder verzweigten Paraffinölen mit 10—30 Kohlenstoffatomen in der Kette in einer Menge von 20—60 Gew.-%, bezogen auf das gesamte Mittel;
— eine dispergierte wässerige Phase (emulgierte Phase);
— ein Puffer, hauptsächlich bestehend aus Milchsäure in einer Menge von 0,1—1,5 Gew.-%, bezogen auf das gesamte Mittel, und Triäthanolamin in einer Menge von 1—2 Gew.-%, bezogen auf das gesamte Mittel, wobei der Puffer den normalen pH-Wert der äußeren Hautgewebeschichten einstellt;
— ein Emulgiersystem in einer Menge von 1—5 Gew.-%, bezogen auf das gesamte Mittel, hauptsächlich bestehend aus Mono- und Diglyceriden von natürlichen höheren Fettsäuren sowie äthoxyliertem Glycerin, das mit Fettsäure verestert ist, gemäß der allgemeinen Formel

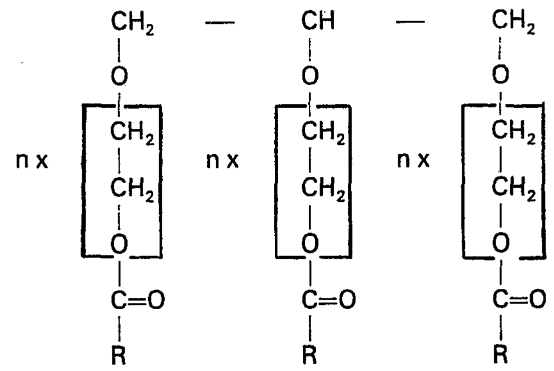

in der n eine Zahl von 20—45, vorzugsweise 30 bis 35, und R einen gesättigten oder ungesättigten Fettsäurerest, der von tierischen oder pflanzlichen Ölen abgeleitet ist, bedeuten, wobei die Reste R in einem Molekül gleiche oder verschiedene Fettsäurereste darstellen, sowie das Verhältnis der Mengen der Mono- und Diglyceride under der äthoxylierten Triglyceride 10—100 Teile Mono- und Diglyceride pro Teil der äthoxylierten Triglyceride beträgt;

— Glycerin in einer Menge von 0,5—5 Gew.-%, bezogen auf das gesamte Mittel;
— Carraghenate, gegebenenfalls modifiziert durch Verestern der Sulfonsäurereste mit Glykol, Propylenglykol und Glycerin, in einer Menge von 0,1—2 Gew.-%, bezogen auf das gesamte Mittel.

2. Handwaschmittel nach Anspruch 1, dadurch gekennzeichnet, daß es einen Stabilisator, z.B. eine System, bestehend aus Montmorilloniten, deren freie Sauerstoffe quartäre Reste tragen, enthält.

3. Handwaschmittel nach Anspruch 1 und 2, dadurch gekennzeichnet, daß es als zusätzliche Bestandteile Allantoin, Propyl-p-hydroxybenzoat und/oder Methyl-p-hydroxybenzoat als Konservierungsmittel, Siliconöl, Oxidationsschutzmittel, ein niederes Alkanol mit 1—4 Kohlenstoffatomen, Duftstoffe und Desinfektionsmittel enthält.

4. Handwaschmittel nach Anspruch 1, dadurch gekennzeichnet, daß die kontinuierliche Ölphase, bestehend aus Fraktionen von gegebenenfalls verzweigtem Paraffinöl mit 12—25 Kohlenstoffatomen, in einer Menge von 20—60, vorzugsweise 25—40, Gew.-%, bezogen auf das gesamte Mittel, vorliegt, der Puffer aus Milschsäure in einer Menge von 1,0 Gew.-%, bezogen auf das Gewicht des gesamten Systems, besteht, während das Triäthanolamin in einer Menge vorliegt, daß ein pH-Wert von 5—6 eingestellt wird, sowie das Verhältnis der Mengen der Mono- und Diglyceride und der äthoxylierten Triglyceride gemäß der angegebenen Formel 25 Teile Mono- und Diglyceride pro Teil äthoxylierte Triglyceride in einem gesamten Prozentsatz von etwa 3 Gew.-%, bezogen auf das Gewicht des gesamten Systems, beträgt, sowie Glycerin in einer Menge von 2 Gew.-%, Carraghenate in einer Menge von 1,0 Gew.-% und eine niederes Alkanol in einer Menge von 0,1—1 Gew.-%, bezogen auf das Gewicht des gesamten Systems, vorliegen.

5. Handwaschmittel nach Anspruch 1 bis 4, dadurch gekennzeichnet, daß es enthält Allantoin in einer Menge von 0,1—2, vorzugsweise etwa 0,2, Gew.-%, bezogen auf das gesamte Gemisch, Montmorillonite in einer Menge von höchstens 2, vorzugsweise 1,0, Gew.-%, bezogen auf das gesamte Gemisch, Konservierungsmittel in Form von Estern von p-Hydroxybenzoesäure in einer Menge von 0,02—0,08, vorzugsweise, 0,05, Gew.-% in der Ölphase und in einer Menge von 0,05—0,2, vorzugsweise, 0,1, Gew.-% in der wässerigen Phase, bezogen auf das Gewicht des gesamten Systems, Siliconöl mit einer Viskosität von 100 cP bis zu einer Menge von 3 Gew.-%, bezogen auf das Gesamtgewicht des Mittels, ein oder mehrere Oxidationsschutzmittel in einer Menge von 0,01—0,03 Gew.-%, Duftstoffe in einer Menge von 0,03 bis 0,3 Gew.-% und Desinfektionsmittel in einer Menge bis zu 1,5 Gew.-%, bezogen auf das Gewicht des gesamten Systems.

6. Handwaschmittel nach Anspruch 1 bis 5, dadurch gekennzeichnet, daß das verwendete Emulgiersystem aus Mono- und Diglyceriden von Ölsäure und äthoxyliertem Glycerin, das mit Ölsäure gemäß der angegebenen Formel verestert ist, in der n eine Zahl von 30 bis 35 bedeutet, besteht.

7. Handwaschmittel nach Anspruch 3, 5 und 6, dadurch gekennzeichnet, daß als Konservierungsmittel in der Ölphase der Propylester der p-Hydroxybenzoesäure in einer Menge von 0,02—0,05 Gew.-% und als Konservierungsmittel in der wässerigen Phase der Methylester der p-Hydroxybenzoesäure in einer Menge von 0,05—0,1 Gew.-%, bezogen auf das gesamte System, vorliegen.

8. Verfahren zur Herstellung eines Handwaschmittels nach einem der Ansprüche 1 bis 7, dadurch gekennzeichnet, daß man die Fett- oder Ölphase mit dem Emulgiersystem mischt, gegebenenfalls nachfolgend das Konservierungsmittel und/oder die Oxidationsschutzmittel und/oder Siliconöl zusetzt, nachfolgend die Montmorillonit-Fraktion bei einer Temperatur von 50—75°C, vorzugsweise 55—65°C, zufügt und geliert, nachfolgend ein niederes Alkanol bis zu einer Menge, die der halben Gewichtsmenge der Montmorillonit-Fraktion entspricht, zugibt, gegebenenfalls nachfolgend das Konservierungsmittel und/oder Oxidationsschutzmittel zusetzt, gegebenenfalls nachfolgend Siliconöl

zugibt, nachfolgend die wässerige Phase einarbeitet, worin alle anderen Bestandteile vorher bei etwa 40°C gelöst worden sind, gegebenenfalls nachfolgend dazwischen während 10—30 Minuten kräftig rührt und nachfolgend homogenisiert, bis eine Größe der dispergierten Teilchen von <5$\mu$, vorzugsweise <3$\mu$, erreicht ist, sowie gegebenenfalls anschließend ein Schleifmittel unter kräftigem Rühren zusetzt.

**Revendications**

1. Composition pour le lavage des mains, caractérisée en ce qu'elle est composée au moins des constituants suivants:

— une phase huileuse continue, consistant principalement en fractions d'huile de paraffine linéaire ou ramifiée dont la chaîne compte 10—30 atomes de carbone, en une quantité de 20—60% en poids, sur base du poids de la composition complète,
— une phase aqueuse dispersée (phase émulsionnée);
— un tampon composé principalement d'acide lactique en quantité de 0,1 à 1,5% en poids, sur base de la composition complète, et de triéthanolamine en une quantité de 1—2% en poids, sur base de la composition complète, le tampon conduisant au pH normal des couches du tissu cutané extérieur,
— un système émulsionnant d'une quantité de 1—5% en poids, sur base de la composition complète, consistant principalement en mono- et diglycérides des acides gras naturels supérieurs et en glycérine éthoxylée estérifiée par des acides gras, de la formule générale suivante:

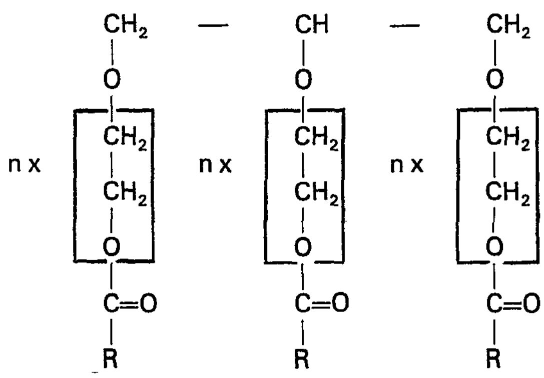

où n représente un nombre de 20 à 45 et de préférence de 30 à 35, tandis que R représente un reste d'acide gras saturé ou insaturé provenant d'huiles animales ou végétales, étant entendu que R représente des restes d'acides gras identiques ou différents dans une molécule, le rapport des quantités des mono- et diglycérides et des triglycérides éthoxylés pouvant varier entre 10 et 100 parties de mono- et diglycérides par partie de triglycérides éthoxylés,

— de la glycérine en une quantité de 0,5—5% en poids, sur base de la composition complète
— des carragheenates, éventuellement modifiés par estérification des restes acide sulfonique par du glycol, du propylèneglycol et du glycérol, en une quantité de 0,1—2% en poids, à calculer sur la composition complète.

2. Composition pour le lavage des mains suivant la revendication 1, caractérisée en ce qu'elle contient des agents stabilisants tels que des systèmes consistant en montmorillonites, dont les sites d'oxygène libres sont occupés par des radicaux quaternaires.

3. Composition pour le lavage des mains suivant les revendications 1 et 2, caractérisée en ce qu'elle contient comme constituants supplémentaires de l'allantoïne, du p-hydroxybenzoate de propyle et/ou du p-hydroxybenzoate de méthyle comme agents de conservation, de l'huile de silicone, des antioxydants, un alcanol inférieur comptant 1 à 4 atomes de carbone, des parfums et des désinfectants.

4. Composition pour le lavage des mains suivant la revendication 1, caractérisée en ce que la phase huileuse continue, consistant en fractions d'huile de paraffine éventuellement ramifiée de 12 à 25 atomes de carbone, est présente en un pourcentage pondéral de 20—60% et de préférence de 25 à 40% du poids de la composition complète, le tampon consiste en acide lactique en une quantité de 1,0% en poids, à calculer sur le poids du système complet, tandis que la triéthanolamine est présente en une quantité établissant un pH de 5—6, étant entendu que le rapport entre la quantité de mono- et diglycérides et des triglycérides éthoxylés de la formule 1 est de 25 parties de mono- et diglycérides par partie de triglycérides éthoxylés, en un pourcentage total à calculer sur le poids du système complet, d-environ 3% en poids, la glycérine est présente en un pourcentage de 2% en poids, les carragheenates en un pourcentage de 1,0% en poids et l'alcanol inférieur en un pourcentage pondéral de 0,1—1%, à calculer sur le poids du système complet.

5. Composition pour le lavage des mains suivant les revendications 1 à 4, caractérisée en ce qu'elle contient de l'allantoïne en quantité de 0,1—2% en poids et de préférence d'environ 0,2% en poids, à calculer sur le poids du mélange complet, des montmorillonites en une quantité d'au maximum 2% en poids et de préférence de 1,0% en poids, à calculer sur le mélange complet, des agents de conservation sous la forme des esters d'acide p-hydroxybenzoïque en une quantité de 0,02—0,08% en poids dans la phase huileuse et de préférence de 0,05% en poids et en une quantité dans la phase aquese de 0,05—0,2% en poids et de préférence de 0,1%, à calculer sur le poids du système complet de l'huile de silicone ayant une viscosité >100 cP (0,1 Pa.s) en quantité s'élevant jusqu'à 3% en poids, à calculer sur le poids total de la composition, un ou plusieurs antioxyants en quantité de 0,01 à 0,03% en poids, des parfums en une quantité de 0,03 à 0,3% en poids et des désinfectants en une quantité s'élevant jusqu'à 1,5% en poids, à calculer sur le poids du système complet.

6. Composition pour le lavage des mains suivant les revendications 1 à 5, caractérisée en ce que le système émulsionnant utilisé est composé de mono- et diglycérides d'acide oléique et de glycérine éthoxylée estérifiée par de l'acide oléique, répondant à la formule 1 où n représente un nombre entre 30 et 35.

7. Composition pour le lavage des mains suivant les revendications 3, 5 et 6, caractérisée en ce que l'ester propylique d'acide p-hydroxybenzoïque est présent comme agent de conservation dans la phase huileuse en quantité de 0,02—0,05% en poids et l'ester méthylique d'acide p-hydroxybenzoïque est présent comme agent de conservation dans la phase aqueuse en quantité de 0,05—0.1% en poids, à calculer sur le système complet.

8. Procédé de préparation d'une composition pour le lavage des mains suivant les revendications 1 à 7 précédentes, caractérisé en ce que la phase grasse ou huileuse est mélangée avec le système émulsionnant, éventuellement ensuite avec l'agent de conservation et/ou les antioxydants et/ou l'huile de silicone, ensuite par addition et gélification de la fraction de montmorillonite à une température de 50—75°C, et de préférence de 55—65°C, ensuite par addition de l'alcanol inférieur en quantité d'au maximum la moitié du poids de la fraction de montmorillonite, éventuellement ensuite par addition de l'agent de conservation et/ou des antioxydants, éventuellement ensuite par addition de l'huile de silicone, ensuite par addition de la phase aqueuse, dans laquelle tous les autres constituants ont été au préalable dissous à environ 40°C, éventuellement ensuite par vive agitation intermédiaire pendant 10—30 minutes, et ensuite par homogénéisation jusqu'à atteindre une dimension des particules dispersées de <5$\mu$m et de préférence de <3$\mu$m, après quoi un abrasif est éventuellement ajouté sous vive agitation.